(19) Europäisches Patentamt

European Patent Office

Office européen des brevets

(11) **EP 1 688 490 A1**

(12) **EUROPEAN PATENT APPLICATION**
published in accordance with Art. 158(3) EPC

(43) Date of publication:
09.08.2006 Bulletin 2006/32

(51) Int Cl.:
*C12N 15/09* (2006.01)  *C12N 1/15* (2006.01)
*C12N 1/19* (2006.01)  *C12N 1/21* (2006.01)
*C12N 5/00* (2006.01)  *C12P 21/02* (2006.01)

(21) Application number: 04793208.2

(22) Date of filing: 29.10.2004

(86) International application number:
**PCT/JP2004/016100**

(87) International publication number:
**WO 2005/042738 (12.05.2005 Gazette 2005/19)**

(84) Designated Contracting States:
**AT BE BG CH CY CZ DE DK EE ES FI FR GB GR HU IE IT LI LU MC NL PL PT RO SE SI SK TR**

(30) Priority: 30.10.2003 JP 2003371004

(71) Applicant: DAIICHI PHARMACEUTICAL CO., LTD.
Chuo-ku,
Tokyo 103-8234 (JP)

(72) Inventors:
• **YOKOTA, Hiroshi,**
**DAIICHI PHARMACEUTICAL CO., LTD**
Edogawa-ku
134-8630 Tokyo (JP)

• **KIKUYA, Eriko,**
**DAIICHI PHARMACEUTICAL CO., LTD**
Ichikawa-shi
Chiba 272-0001 (JP)

(74) Representative: **Forstmeyer, Dietmar et al**
**BOETERS & LIECK**
Oberanger 32
80331 München (DE)

(54) **DOSE-DEPENDENT PROMOTER ORIGINATING IN HUMANS**

(57) It was found that a novel DNA consisting of a nucleotide sequence as set forth in SEQ ID NO: 1 having one sequence (SEQ ID NO: 2) unit, has a function of an initiator or a promoter. Therefore, it is provided a novel DNA consisting of a nucleotide sequence as set forth in SEQ ID NO: 1; a DNA in which a 5'-end of a DNA consisting of a nucleotide sequence as set forth in SEQ ID NO: 1 is added to a 3'-end of a DNA having one or more of the sequence (SEQ ID NO: 2) units; the above DNA further containing a structural gene; a vector having the above DNA inserted therein; a transformant having the vector transfected therein; a method of regulating gene expression or a method of producing a protein using one member selected from the above DNAs, vector and transformant; and a reagent kit containing one member selected from the above DNAs, vector and transformant.

EP 1 688 490 A1

**Description**

**TECHNICAL FIELD**

The present invention relates to a human-derived DNA having transcriptional activity and the use thereof. More particularly, the present invention relates to the following: a human-derived DNA consisting of one specific sequence or a conjugate of a plurality of the specific sequences ligated to each other, and can enhance transcriptional activity in a manner dependent on the number of the sequences ligated; a DNA construct containing the DNA and so on. The present invention also relates to a vector containing the aforementioned DNA or the aforementioned DNA construct, and to a transformant having the vector transfected therein. The present invention also relates to a method of preparing the aforementioned DNA. Further, the present invention relates to a method of regulating the amount of expression of a gene, which is characterized by using the aforementioned DNA, the aforementioned DNA construct or the aforementioned vector. Furthermore, the present invention relates to a method of producing a protein comprising using the aforementioned DNA, the aforementioned DNA construct, the aforementioned vector or the aforementioned transformant. The present invention also relates to a reagent kit containing at least one member selected from the aforementioned DNA, the aforementioned DNA construct, the aforementioned vector and the aforementioned transformant.

**BACKGROUND ART**

Genetic information contained in a DNA is normally transcribed into a messenger RNA (mRNA) by a RNA polymerase, and subsequently translated into an amino acid sequence of a protein in accordance with the mRNA information. In prokaryotes, the fundamental transcription can be performed by a RNA polymerase alone. In eukaryotes, the initiation of a transcription reaction requires a series of proteins called transcription factors in addition to a RNA polymerase.

Transcription factors are assembled sequentially at a region called a "promoter" on a gene, and forms a transcription initiation complex together with a RNA polymerase to initiate a transcription reaction. The transcription reaction is regulated by transcription control elements such as a promoter, an enhancer or a suppressor, and so on.

An initiator or a promoter which is an apparatus for regulating gene expression has a function that determines the transcription initiation site of a gene and directly regulates the frequency of transcription initiation. Promoters of most structural genes contain a TATA box, a CCAAT box and/or a GC rich region as an essential element. There are also some promoters that have an initiator sequence at a transcription initiation site instead of a TATA box. The initiator sequence can be a sequence such as YYAN(A/T)YY, where Y can be C or T, and N can be A or T or C or G

The promoter is an important element for enhancing expression efficiency in expressing a gene that encodes a useful protein by gene manipulation techniques. In particular, in an expression of an animal-derived gene by using an animal cell as a host cell, selection of a promoter provides a significant influence on the expression efficiency. An SV40 promoter, a LTR promoter, a SR$\alpha$ promoter, a cytomegalovirus promoter, an actin promoter and the like are known animal cell promoters and are frequently used.

References cited in the specification are:

Non-Patent Document 1: Sambrook et al., "Molecular Cloning, A Laboratory Manual, Second Edition", 1989, Cold Spring Harbor Laboratory.
Non-Patent Document 2: "EMBO Journal", 1982, Vol. 1, p. 841 - 845.

**DISCLOSURE OF THE INVENTION**

[PROBLEMS TO BE SOLVED BY THE INVENTION]

An object of the present invention is to obtain a novel DNA having a function of a promoter or an initiator which is an apparatus for regulating gene expression, and to use it for gene expression.

[MEANS FOR SOLVING THE PROBLEMS]

The present inventors have concentrated their efforts to meet the aforementioned object and have discovered a region that is present in the vicinity of a site that is considered a transcription initiation site of a gene referred to as "synaptotagmin 11" that contains repeated 33-mer DNAs, each of which has a TATA box, an initiator sequence and a binding site for transcription factor SP-1, which can be characteristics of an initiator or a promoter region, by performing in-silico analysis and the like. The present inventors have found that a DNA in which a DNA consisting of TCC is added to the 3'-end of the 33-mer DNA or of a DNA consisting of a plurality of the 33-mer DNAs that are ligated sequentially to each other, has a transcriptional activity, and furthermore, that the transcriptional activity is enhanced in a manner dependent on the number of the 33-mer DNAs ligated, thereby to complete the present invention.

In various embodiments, the present invention relates to a DNA consisting of a nucleotide sequence as set forth in SEQ ID NO: 1.

The present invention also relates to a DNA in which a 5'-end of a DNA consisting of a nucleotide sequence as set forth in SEQ ID NO: 1 is added to a 3'-end of a DNA in which one or more of a DNA consisting of a nucleotide sequence as set forth in SEQ ID NO: 2 are linked to each other, wherein the link in the DNA in which one or more of the DNA consisting of the nucleotide sequence as set forth in SEQ ID NO: 2 are linked to each other is such that the 3'-end of a DNA consisting of the nucleotide sequence as set forth in SEQ ID NO: 2 is adjacent to the 5'-end of DNA located on that 3'-side.

The present invention further relates to a DNA consisting of a nucleotide sequence as set forth in any one of SEQ ID NOS: 3 to 6.

The present invention still further relates to any one of the aforementioned DNAs, which has a transcriptional activity, wherein the nucleotide sequence as set forth in SEQ ID NO: 2 is a sequence unit and the transcriptional activity is enhanced in a manner dependent on the number of the sequence units contained in the DNA.

The present invention also relates to any one of the aforementioned DNAs, which has a transcriptional activity, wherein the nucleotide sequence as set forth in SEQ ID NO: 2 is a sequence unit and the transcriptional activity is enhanced in a manner dependent on a number of from one to seven of the sequence units contained in the DNA.

The present invention further relates to an apparatus for regulating gene expression, comprising any one of the aforementioned DNAs.

The present invention still further relates to a DNA comprising a structural gene and any one of the aforementioned DNAs, wherein any one of the aforementioned isolated DNAs is positioned so as to enable the expression of the structural gene.

The present invention also relates to a vector comprising any one of the aforementioned DNAs.

The present invention further relates to a vector comprising any one of the aforementioned DNAs and a DNA having an enhancer function.

The present invention still further relates to the aforementioned vector comprising a structural gene, wherein the any one of the aforementioned DNAs is positioned so as to enable the expression of the structural gene.

The present invention also relates to any one of the aforementioned vectors, wherein the vector is a mammalian expression vector.

The present invention further relates to any one of the aforementioned vectors, wherein the vector is a virus vector.

The present invention still further relates to any one of the aforementioned vectors, wherein the vector is a gene therapy vector.

The present invention also relates to a transformant which is transformed with any one of the aforementioned vectors.

The present invention further relates to the aforementioned transformant, wherein the vector is transfected into a mammalian cell.

The present invention still further relates to a method of preparing any one of the aforementioned DNAs, wherein the method comprises reacting a DNA consisting of a nucleotide sequence as set forth in SEQ ID NO: 2 with a DNA consisting of a nucleotide sequence as set forth in SEQ ID NO: 7 to form double-stranded DNAs, then ligating the prepared double-stranded DNAs to produce a conjugate, and then carrying out a polymerase chain reaction using the conjugate as a template.

The present invention also relates to the aforementioned method of preparing the DNA, wherein the polymerase chain reaction is carried out using DNAs consisting of either of nucleotide sequences as set forth in SEQ ID NOS: 8and 9.

The present invention further relates to a method of regulating an amount of expression of a gene, wherein the method comprises using any one of the aforementioned DNAs, or any one of the aforementioned vectors.

The present invention still further relates to a method of producing a protein, wherein the method comprises using any one of the aforementioned DNAs, any one of the aforementioned vectors, or the aforementioned transformant.

The present invention also relates to a reagent kit, comprising at least one member selected from any one of the aforementioned DNAs, any one of the aforementioned vectors, and the aforementioned transformant.

[ADVANTAGES OF THE INVENTION]

According to various embodiments, a DNA, in which a DNA consisting of TCC is linked to a 3'-end of a 33-mer DNA (SEQ ID NO: 2) or of a DNA consisting of a plurality of the 33-mer DNAs that are ligated sequentially to each other, was found to have a transcriptional activity. Furthermore, the transcriptional activity is enhanced in a manner dependent on the number of the 33-mer DNAs contained in the aforementioned DNA.

Therefore, the DNA, in which a DNA consisting of TCC is linked to a 3'-end of a 33-mer DNA (SEQ ID NO: 2) or of a DNA consisting of a plurality of the 33-mer DNAs that are ligated sequentially to each other, can be favorably used in a gene expression method. Further, by increasing or decreasing the number of the 33-mer DNA (SEQ ID NO: 2) units ligated, artificial regulation of the amount of expression of a protein is enabled. The present invention can thereby provide an effective means for elucidating disorders caused by multiple factors, such as lifestyle-related diseases. More specifically, regulation of subtle quantitative variations in a plurality of target proteins that are pathology-related can be achieved using the same vector having a structural unit consisting of the 33-mer DNA (SEQ ID NO: 2), and therefore it is possible to provide an effective means for elucidating a pathology attributable to an abnormality of the protein. The present invention can also be utilized for producing a useful protein or for an expression vector for gene therapy, and so on.

## BRIEF DESCRIPTION OF THE *DRAWINGS

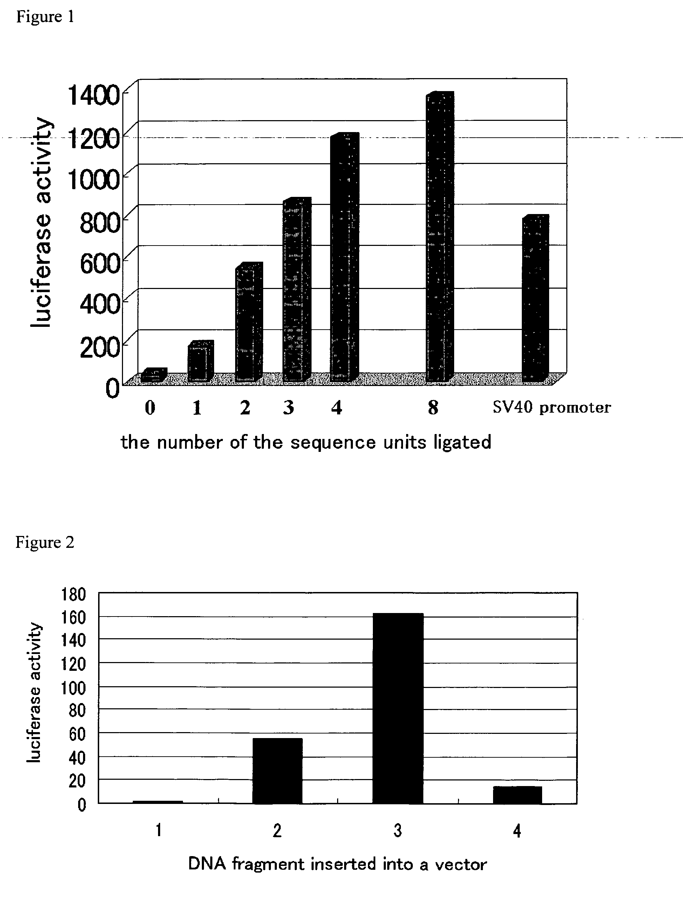

Figure 1 shows that in a cell lysate of COS-1 cells, which were transfected with an expression vector that contains DNA consisting of any one of the nucleotide sequences as set forth in SEQ ID NO: 1, SEQ ID NO: 3, SEQ ID NO: 4, SEQ ID NO: 5 and SEQ ID NO: 6 (the number of the sequence (SEQ ID NO: 2) units ligated are 1, 2, 3, 4 and 8, respectively), a luciferase gene downstream of the DNA, and an SV40 enhancer, luciferase activity increased quantitatively in proportion to the number of the sequence (SEQ ID NO: 2) units ligated. In the figure, the phrase "SV 40 promoter" refers to a cell lysate of COS-1 cells which were transfected with an expression vector that contains an SV40 promoter instead of the aforementioned DNA.
(Example 1)
Figure 2 shows that in a cell lysate of COS-1 cells, which were transfected with an expression vector that contains DNA consisting of any one of the nucleotide sequences as set forth in SEQ ID NO: 3, SEQ ID NO: 13 and SEQ ID NO: 14 (the number of the sequence (SEQ ID NO: 2) units ligated are 2, 2, and 3, respectively) and a luciferase gene downstream of the DNA, but does not contain an SV40 enhancer, luciferase activity increased quantitatively in proportion to the number of the sequence (SEQ ID NO: 2) units ligated. In the figure, reference numeral 1 is for the result obtained using only a pGL-Basic vector, reference numeral 2 is for the result obtained using a vector that contains the DNA consisting of the nucleotide sequence as set forth in SEQ ID NO: 13, reference numeral 3 is for the result obtained using a vector that contains the DNA consisting of the nucleotide sequence as set forth in SEQ ID NO: 14, and reference numeral 4 is for the result obtained using a vector that contains the DNA consisting of the nucleotide sequence as set forth in SEQ ID NO: 3. (Example 2)

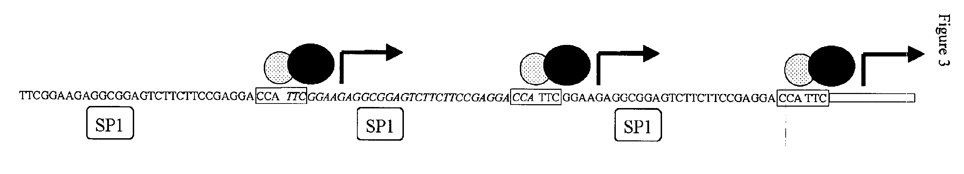

Figure 3 is a schematic diagram showing a presumed mechanism whereby the transcriptional activity of the DNA of the present invention is enhanced in a manner dependent on the number of the sequence (SEQ ID NO: 2) units ligated. The mechanism is ascribed to the fact that a TATA box or an initiator sequence which is a binding site for transcription initiation-related factors such as RNA polymerase is formed in the ligation site within the conjugate and increases in number in accordance with the number of the sequence (SEQ ID NO: 2) units ligated. The present DNAs have nucleotide sequences that contain a consensus GC box which is a binding site for transcription factor SP-1. Therefore the binding sites of SP-1 similarly increase in accordance with the number of the sequence (SEQ ID NO: 2) units ligated.

## DETAILED DESCRIPTION OF THE INVENTION

The present invention was accomplished by finding a region that is present in the vicinity of a site that is considered a transcription initiation site of a gene referred to as "synaptotagmin 11" and contains repeated 33-mer DNAs each of which has a TATA box, an initiator sequence and a binding site for transcription factor SP-1, which are characteristics of an initiator or a promoter region, and finding the functions of the DNA.

In the present specification, the above 33-mer DNA (SEQ ID NO: 2) is referred to as a "sequence unit". Further, a DNA in which a plurality of DNAs each of which consists of the sequence units sequentially linked to each other and joined at their respective ends is referred to as a "conjugate", and the number of the sequence units within the conjugate is referred to as "the number of the sequence units ligated".

An aspect of the present invention relates to a DNA comprising one of or a plurality of the sequence (SEQ ID NO: 2) units. A DNA comprising one sequence (SEQ ID NO: 2) unit can be preferably represented by a DNA consisting of a nucleotide sequence as set forth in SEQ ID NO: 1.

An example of a DNA comprising a plurality of the sequence (SEQ ID NO: 2) units is a DNA in which a 5'-end of a DNA consisting of a nucleotide sequence as set forth in SEQ ID NO: 1 is added to a 3'-end of a DNA consisting of one or more of the sequence (SEQ ID NO: 2) units. The number of the sequence (SEQ ID NO: 2) units added to the DNA consisting of the nucleotide sequence as set forth in SEQ ID NO: 1 is preferably from 1 to 7. When two or more of the sequence (SEQ ID NO: 2) units are added, it is preferable that the sequence (SEQ ID NO: 2) units are sequentially linked such that each 3'-end thereof is linked to the 5'-end of the adjacent downstream DNA consisting of the nucleotide sequence as set forth in SEQ ID NO: 2. Alternatively, a DNA comprising a plurality of the sequence (SEQ ID NO: 2) units can be a DNA consisting of any one of the nucleotide sequences as set forth in SEQ ID NOS: 3 to 6.

The DNA consisting of the nucleotide sequence as set forth in SEQ ID NO: 1 is a DNA in which a DNA consisting of TCC is added to the 3'-end of the above 33-mer DNA (SEQ ID NO: 2), such that it has one sequence unit that is defmed in the present specification. A DNA in which one sequence (SEQ ID NO: 2) unit is added in the above manner to the DNA consisting the nucleotide sequence as set forth in SEQ ID NO: 1 therefore has two sequence (SEQ ID NO: 2) units. Thus, the number of sequence units ligated in this DNA is two. Thus, in the present specification, where a DNA in which "n" number of the sequence (SEQ ID NO: 2) units are added in the above manner to the DNA consisting of the nucleotide sequence as set forth in SEQ ID NO: 1, the DNA therefore has "n+1" number of the sequence (SEQ ID NO: 2) units. Thus, the number of the sequence units ligated in this DNA is "n+1 ".

The DNA of the present invention has a transcriptional activity, and is characterized in that the transcriptional activity increases in a manner dependent on the number of the sequence (SEQ ID NO: 2) units ligated. For example, in a cell lysate of COS-1 cells transfected with an expression vector that comprises DNA consisting of any one of the nucleotide sequences as set forth in SEQ ID NOS: 1, 3, 4, 5, and 6 (the number of the sequence units ligated are 1, 2, 3, 4 and 8, respectively), a luciferase gene downstream of the DNA, and further an SV40 enhancer downstream thereof, luciferase activity increased quantitatively in proportion to the number of the sequence units ligated contained in the DNA (see Example 1 and Figure 1). Further, the transcriptional activity of the DNA in which the number of the sequence units ligated is three was the same as or greater than the transcriptional activity of an SV40 promoter (see Example 1 and Figure 1). However, the transcriptional activity of the present DNA shown in the result of a similar study using an expression vector that did not contain an SV40 enhancer was lower than that shown in the result obtained using an expression vector containing an SV40 enhancer, but was enhanced in proportion to the number of the sequence (SEQ ID NO: 2) units ligated (see Example 2 and Figure 2). Based on these results, it was found that i) the DNA of the present invention has a transcriptional activity that is the same as or greater than that of an SV40 promoter, ii) the transcriptional activity is enhanced in a manner dependent on the number of the sequence (SEQ ID NO: 2) units ligated, and iii) the transcriptional activity of the DNA is significantly increased further by the influence of an SV 40 enhancer.

The mechanism whereby the transcriptional activity of the DNA of the present invention is enhanced in a manner dependent on the number of the sequence (SEQ ID NO: 2) units ligated is not clear. However, it is postulated that the ligation of the units provides a ligation site in which an initiator sequence or a sequence consisting of CCATTC that is considered as a TATA box, is formed, which is a binding site for transcription initiation-related factors such as a polymerase, and the number of the initiator sequence or TATA box increases in accordance with the number of units ligated which results in the initiation of transcription reaction one after the other, and thereby transcription of the downstream gene is enhanced (Figure 3).

The DNA of the present invention has a transcriptional activity, a so-called initiator/promoter activity. Therefore the DNA can be used as an apparatus for regulating gene expression, such as an initiator or a promoter. Among the known initiators and promoters, few DNAs have been reported to have a ligation structure comprising a specific sequence like that of the present DNA and to have an initiator/promoter activity which increases in a manner dependent on the number of the specific sequence units ligated. As used in the present specification, the phrase "initiator/promoter activity" refers to a function and an ability to allow production of the gene product of a structural gene inside or outside a host, when the gene linked downstream of the initiator or promoter is transfected into the host. Since the present DNA has this activity, the DNA can be used to regulate gene expression. Evaluation of initiator/promoter activity can generally be carried out by linking a reporter gene to and downstream of an initiator or a promoter so as to enable the expression of the gene followed by transfecting it into a host and measuring the amount of expression of the gene. A reporter gene that is preferably used is a gene that encodes a protein which can be easily quantitatively measured, for example a gene encoding an enzyme protein such as a luciferase gene. Such an evaluation based on the strength or weakness of the expression of the reporter gene can determine the strength or weakness of the initiator/promoter activity.

The DNA of the present invention can be prepared by chemical synthesis using a known method of synthesizing nucleic acids.

Preparation of a DNA consisting of the nucleotide sequence as set forth in SEQ ID NO: 1 or SEQ ID NO: 2 can be performed using a known gene manipulation technique. For example, the DNA can be prepared by a method that comprises constructing a genomic DNA library for use as a template, and preparing a primer based on the nucleotide sequence as set forth in SEQ ID NO: 1 or SEQ ID NO: 2 to amplify the DNA, and then utilizing a polymerase chain reaction (PCR).

The DNA of the present invention can also be prepared by i) reacting a DNA consisting of the nucleotide sequence as set forth in SEQ ID NO: 2 with a DNA consisting of the nucleotide sequence as set forth in SEQ ID NO: 7 under appropriate conditions to form a double-stranded DNA (hereinafter referred to as "adapter"); ii) then ligating a plurality of the prepared adapters by a known ligation technique to prepare an adapter conjugate; iii) then carrying out PCR using the adapter conjugate which is used as a template and a primer that was prepared based on the nucleotide sequence as set forth in SEQ ID NO: 1; and iv) inserting the obtained PCR amplification product into a suitable vector, using the vector for cloning the product in a host such as *Escherichia coli,* and then selecting clones having the desired number of conjugates to obtain the DNA from the clones.

An example of the temperature cycle for forming an adapter is heating at 90°C for 1 min; cooling from 70°C to 40°C over 1 h; incubating at 40°C for 15 min; followed by further cooling to 4°C over 1 h. Ligation of the adapter can be conveniently performed using a commercially available DNA ligation kit or the like.

A PCR primer can be obtained by designing based on the nucleotide sequence set forth in SEQ ID NO: 1 to amplify the desired DNA, and chemically synthesizing the primer by a known method. For example, when adding restriction enzyme sites to the 5'- and 3'-ends of the DNA in order to facilitate transfection of the DNA of the present invention into a vector, a designed primer in which a desired restriction enzyme site is added to the above primer can be chemically synthesized and used for carrying out PCR. More specifically, for example, preparation of a DNA that has a MluI site added to its 5' end and a BglII site added to its 3' end as restriction enzyme sites can be carried out by PCR using a DNA consisting of the nucleotide sequence as set forth in SEQ ID NO: 8 and a DNA consisting of the nucleotide sequence as set forth in SEQ ID NO: 9 as primers. These prepared DNAs having restriction enzyme sites can then be digested with suitable restriction enzymes and thereafter cloned into multicloning sites of a vector that was treated with the same restriction enzymes.

A method of preparing the DNA of the present invention is not limited to these instances, and any known methods such as a common gene manipulation technique or a chemical synthesis technique can also be used.

Another aspect of the present invention relates to a DNA construct containing a structural gene and the DNA of the present invention, in which the DNA is positioned upstream of the structural gene so as to enable the expression of the structural gene. As used herein, the phrase "enable the expression of the structural gene" refers to the fact that the gene is under control of an initiator/promoter activity exerted by the DNA. The DNA construct can be prepared by any known gene manipulation techniques using the DNA of the present invention and a desired structural gene. The structural gene can be obtained, for example, by constructing a human-derived cDNA library in the case of using a human structural gene, designing a primer for amplifying the desired gene based on the nucleotide sequence of the gene and synthesizing it, and then employing a genetic manipulation method such as PCR.

A further aspect of the present invention relates to a vector containing the DNA of the present invention. The vector of the present invention can be obtained by inserting the present DNA into a suitable vector. The vector is not particularly limited as long as it is replicable in a host. A desired vector can be selected for use from known vectors according to the desired purpose. Since the present DNA is a human-derived DNA, the use of a mammalian expression vector is preferable for regulating the expression of a structural gene by utilizing the transcriptional activity of the DNA. In the case of gene therapy or the like, an animal virus vector such as a retrovirus or a vaccinia virus or the like can be used. Other virus vectors including a plant virus vector such as a cauliflower mosaic virus (CaMV) can also be used as the vector of the present invention. As for the method of inserting the present DNA into a vector, a method can be used in which the DNA to be inserted is first cleaved with a suitable restriction enzyme and then inserted into a restriction enzyme site or a multicloning site of a suitable vector to integrate the DNA into the vector. However, the method is not limited thereto, and any known gene manipulation methods can be used.

A vector of the present invention can be a vector containing a structural gene and the DNA of the present invention. For example, the DNA is preferably positioned upstream of the structural gene so as to enable the expression of the structural gene. In order to link the DNA to and position it upstream of the structural gene, a DNA ligase or a homopolymer method can be used. If using DNA ligase for linking, the DNA and the structural gene, both of which have an identical restriction enzyme site, can be linked to each other by digesting them with the restriction enzyme followed by adding DNA ligase in accordance with a method described in the literature (Non-Patent Literature 1). The DNA and the structural gene which do not have an identical restriction enzyme site can be linked to each other by treating them with a T4 DNA polymerase to form blunt ends followed by treating with a DNA ligase in the same manner as above.

Any structural gene can be used as long as it can be expressed by using the DNA or vector of the present invention. For example, a structural gene may include a gene derived from mammals, a gene derived from microorganisms such as a bacterium, yeast, actinomycete, fungus, ascomycete or basidiomycete, or a gene derived from organisms such as a plant or an insect. Preferably, the gene is a gene derived from mammals.

The transcriptional activity of the DNA of the present invention was further enhanced by the influence of an enhancer, which indicates that the above vector to which a DNA having a function as an enhancer is linked to, can further enhance the expression of the structural gene. As used herein, the term "enhancer" refers to a sequence that significantly promotes the transcription from an initiator or a promoter. A DNA having a function as an enhancer may be positioned at any position of the 5' upstream side or 3' downstream side of the present DNA or structural gene. An example of such a DNA is an SV40 enhancer. The vector can also contain, as desired, a poly(A) addition signal, and a DNA having a function such as a selective marker or a terminator. As necessary, the vector can also have an origin of replication. As a selective marker, for example, a dihydrofolate reductase gene, an ampicillin-resistant gene, a neomycin-resistant gene and so on can be used.

A further aspect of the present invention relates to a transformant transfected with the DNA or the DNA construct of the present invention. Transfection with the present DNA or the present DNA construct can be carried out using the vector containing the DNA or the DNA construct. A method of transfection using the vector is carried out by using any known gene manipulation techniques. For example, methods such as electroporation, the calcium phosphate technique, and lipofection can be used. As a method carried out without using a vector, for example, a method described in the literature (Non-Patent Literature 2) or the like can be employed.

As a host cell, a mammalian cell is preferably used. More preferably, a cultured mammalian cell can be used. For example, cultured human cells such as FL cells or HeLa cells, and nonhuman mammalian cells including cultured monkey cells such as COS cells or Vero cells, hamster cultured cells such as CHO cells or BHK cells, cultured rat cells such as GH3 cells, and cultured mouse cells such as L cells can be used. However, a host is not limited thereto, and any known host cells that are used in expression of a structural gene or production of a protein can be used.

A further aspect of the present invention relates to a method of regulating the amount of gene expression, comprising using the DNA, the DNA construct or the vector of the present invention. The present DNA has a transcriptional activity that is characteristically enhanced in a manner dependent on the number of the sequence (SEQ ID NO: 2) units ligated in the present DNA. Such a DNA can be used to regulate the amount of expression of a desired gene. More specifically, changing the number of the sequence (SEQ ID NO: 2) units ligated in the DNA can allow artificial regulation of gene expression. For example, a DNA containing a larger number of the sequence (SEQ ID NO: 2) units ligated has a higher transcriptional activity. Therefore use of such a DNA in conducting the expression of a structural gene provides an increasing amount of expression of the gene, which results in an increasing amount of protein production. In contrast, a DNA containing a smaller number of the sequence (SEQ ID NO: 2) units ligated has a lower transcriptional activity. Therefore, the expression of the gene can also be low. Regulation of the amount of gene expression can be similarly carried out using the DNA construct or vector of the present invention.

The method of regulating the amount of gene expression of the present invention allows, for example, elucidation of disorders caused by multiple factors such as lifestyle-related diseases and the like. More specifically, regulation of subtle quantitative variations in various target proteins that are pathology-related can be achieved by using, for example, the same vector having a sequence (SEQ ID NO: 2) unit. Therefore, it is possible to provide an effective means for elucidating pathology attributable to an abnormality of the protein.

The DNA, DNA construct, vector or transformant of the present invention can also be used in a method of producing a protein. For example, a protein can be produced by an ordinary culturing method using a transformant that is obtained by transfecting the vector or a DNA construct in which a desired structural gene was positioned downstream of the present DNA into a host cell by a known method, and then collecting the protein that is encoded by the structural gene, from the obtained culture. Various common culture mediums can be suitably selected and used according to the host cell which was transfected with the vector. In addition, culturing can also be performed under conditions that are suitable for growth of the host cell. When the desired protein is produced inside the cell of the transformant, the protein of interest can be extracted by disrupting the cell. When the desired protein is produced outside the cell, the culture medium can be used as it is, or the cell can be removed by centrifugation or the like to obtain the protein.

The DNA, DNA construct or vector of the present invention can regulate expression of a desired gene, and therefore can be used in gene therapy. For example, they can be applied to gene therapy where a disorder is attributable to a lack or insufficiency of expression of a specific gene. Furthermore, an animal cell that has the DNA, DNA construct or vector transfected therein, preferably such as a human cell, can also be used for gene therapy.

When using for gene therapy, the DNA, DNA construct, vector or transformant of the present invention can be applied to both an in vivo method in which they are introduced directly into a body, and an ex vivo method in which a target cell is first extracted from the body of the patient to introduce a gene into the cell outside the body and thereafter returned to the body of the patient. The in vivo method is more preferably used. The target tissue and target cell for introducing the gene can be suitably selected in accordance with the object of the gene therapy for treatment. Examples of a target cell, but not limited to, are lymphoid cells, fibroblasts, hepatic cells and hematopoietic stem cells.

For a method of transfecting a gene into a body or into a cell, either a non-viral transfection method or a transfection method using a virus vector can be applied. A non-viral transfection method is more preferable for use in comparison to a transfection method that uses a virus vector, since it is superior in terms of safety and convenience in addition to being inexpensive. Both transfection methods can be implemented in accordance to various methods used in gene therapy.

For use in gene therapy, the DNA, DNA construct, vector or transformant is preferably prepared in general as an injection formulation, a drip injection formulation or a liposome formulation. It can also be prepared in a form that allows administration thereof together with a substance that enhances the efficiency of gene transfer, such as a protamine. Administration can be performed once a day or can be divided into several times a day. Alternatively, intermittent administration at an interval of one day to several weeks can be employed.

The DNA, DNA construct, vector and transformant of the present invention can each be used independently as a reagent or the like. Further, a reagent kit containing at least one member selected from the DNA, DNA construct, vector and transformant is also included in the scope of the present invention. The reagent or the reagent kit can contain a substance such as a buffer solution, a salt, a stabilizer and/or an antiseptic agent.

Hereinafter, the present invention is explained more particularly, but is not limited to, the following examples.

Example 1

Production of the 33-mer DNA conjugate
In the vicinity of a site that is considered as a transcription initiation site of a gene referred to as synaptotagmin 11, a region is present which contains a 33-mer DNA (SEQ ID NO: 2) repeats each of which has a TATA box, an initiator sequence and a binding site of transcription factor SP-1, which are characteristics of an initiator or promoter region. The present inventors focused on this region and prepared conjugates that had one or more of the 33-mer DNA (SEQ ID NO: 2) units. Hereinafter, the 33-mer DNA (SEQ ID NO: 2) may be referred to as a "sequence unit."
Synthetic oligonucleotides, 33-mer Uni. (SEQ ID NO: 2) and 33-mer Rev. (SEQ ID NO: 7), were used to prepare a double-stranded DNA fragment (hereinafter, referred to as "adapter") by reacting them with each other under the following temperature cycle: heating at 90°C for 1 min; cooling from 70°C to 40°C over 1 h; incubation at 40°C for 15 min; followed by further cooling to 4°C over 1 h. The 5'-ends of the adapters were phosphorylated with a T4 polynucleotide kinase (Takara Co., Ltd) to prepare an adapter conjugate by ligating two or more of the adapters using DNA Ligation kit (Takara Co., Ltd).
33-mer Uni.: 5'-TTC GGA AGA GGC GGA GTC TTC TTC CGA GGA CCA3' (SEQ ID NO: 2)
33-mer Rev.: 5'-GAA TGG TCC TCG GAA GAA GAC TCC GCC TCT TCC3' (SEQ ID NO: 7)
To the adapter conjugate, restriction enzyme sites were added to make it easy to insert it into a vector (pGL3-Enhancer vector, Promega). More specifically, restriction enzyme sites were added to the adapter conjugates by carrying out PCR using the prepared adapter conjugate as a template and MluI 33-mer Uni. (SEQ ID NO: 8) and BglII R3 Long (SEQ ID NO: 9) as primers. The adapter conjugate with the restriction enzyme sites was subjected to restriction enzyme treatment for 1 h at 37°C with MluI (Takara) and BglII (Takara), and then used for insertion into a vector.
MluI 33-mer Uni.: 5'-CGA CGC GTT TCG GAA GAG GCG GAG TC3' (SEQ ID NO: 8)
BglII R3 Long: 5'-GGA GAT CTG AAT GGT CCT CGG AAG AAG AC3' (SEQ ID NO: 9)
Preparation of vector containing the 33-mer DNA conjugate
The vector used in this example was a pGL3-Enhancer vector (Promega). The pGL3-Enhancer vector was subjected to a restriction enzyme treatment with MluI (Takara) and BglII (Takara) for 1 h at 37°C, and then treated three times with alkaline phosphatase (Takara) at 56°C for 1 h for dephosphorylation. The adapter conjugate, after treating with the restriction enzymes as described above, was inserted into a pGL3-Enhancer vector/MluI/BglII/BAP×3 using DNA Ligation kit, and then used for transformation in *Escherichia coli* (DH5α, Invitrogen Corp.). The sequence of the 33-mer DNA conjugates that was inserted into the vectors and the number of the sequence (SEQ ID NO: 2) units ligated that was contained in the conjugates were examined using the ABI PRISM 377 DNA Sequencer (Applied Biosystems). Finally, it was possible to prepare a vector in which a conjugate having 1, 2, 3, 4 or 8 of the sequence (SEQ ID NO: 2) units was positioned upstream of the luciferase gene in a pGL3-Enhancer vector. The DNA having 1, 2, 3, 4 or 8 of the sequence (SEQ ID NO: 2) units ligated that was inserted into the vectors is set forth in SEQ ID NO: 1, SEQ ID NO: 3, SEQ ID NO: 4, SEQ ID NO: 5 or SEQ ID NO: 6, respectively.
Measurement of transcriptional activity of the 33-mer DNA conjugates
Measurement of the transcriptional activity of the 33-mer DNA conjugates was carried out using the Dual-Luciferase Reporter Assay System (Promega) that uses firefly luciferase as a reporter enzyme and that employs luciferase activity as an indicator.
The above pGL3-Enhancer vector that contains DNA consisting of any one of the nucleotide sequences as set forth in SEQ ID NO: 1, SEQ ID NO: 3, SEQ ID NO: 4, SEQ ID NO: 5 and SEQ ID NO: 6, was used as a test sample. The DNAs, each consisting of any one of the nucleotide sequences as set forth in SEQ ID NO: 1, SEQ ID NO: 3, SEQ ID NO: 4, SEQ ID NO: 5 and SEQ ID NO: 6, can have 1, 2, 3, 4 or 8 of the sequence (SEQ ID NO: 2) units ligated, respectively. An empty pGL3-Enhancer vector was used as a negative control, and a pGL3-Control vector (Promega) containing a SV 40 promoter and an SV40 enhancer was used as a positive control. Simultaneously, to correct variations in the transfection efficiency, a phRL-TK vector having the Renilla luciferase gene as a reporter enzyme was used as an internal control vector.
The vectors were transfected into COS-1 cells that were cultured in DMEM (GIBCO) containing 10% fetal bovine serum (FBS, GIBCO). Transfection was performed according to a known method using a TransFast Reagent (Promega). First, the COS-1 cells were seeded onto 24-well plates (Nunclon Surface; Nunc) ($5 \times 10^4$ cell/ml, 1 ml/well). The next day, 500 ng/well of the test sample and 500 pg/well of a phRL-TK vector were added to 200 $\mu$l/well of a fresh culture medium without FBS and then mixed. The mixture was further added with 1.5 $\mu$l/well of the TransFast Reagent and then well mixed, and then left to stand for 10 min at room temperature. After removing the medium from the cultured cells, which were seeded the previous day, the above mixture was added to the wells to culture at 37°C for 1 h, followed by adding with a further 1 ml of fresh medium containing FBS to culture at 37°C for 24 h. Thereafter, the culture supernatant was removed and a cell lysate was prepared using a Passive Lysis Buffer (including in the kit). The cell lysate was also prepared in a similar manner using a negative control and a positive control instead of the test sample.

Measurement of the luciferase activity of each cell lysate was conducted using a Micro Lumat Plus (Perkin-Elmer). The luciferase activity was shown as a value calculated with the following formula based on the obtained measurement values.

Formula 1:

$$\text{Ratio} = [\text{measurement value of test sample (luminescence intensity of firefly luciferase)}]$$

$$/ [\text{measurement value of internal control vector (luminescence intensity of renilla luciferase)}]$$

As shown in Figure 1, the luciferase activity increased quantitatively in proportion to the number of the sequence (SEQ ID NO: 2) units ligated. It was thus found that the DNA consisting of any one of the nucleotide sequences as set forth in SEQ ID NO: 1, SEQ ID NO: 3, SEQ ID NO: 4, SEQ ID NO: 5 and SEQ ID NO: 6 has a transcriptional activity to enable the expression of a gene positioned downstream of the DNA, and that the transcriptional activity is enhanced in a manner dependent on the number of the sequence units ligated. It was also found that the amount of luciferase expression when using the DNA consisting of any one of the nucleotide sequences as set forth in SEQ ID NO: 4, SEQ ID NO: 5 and SEQ ID NO: 6, was equivalent to or greater than when using an SV40 promoter, indicating that the DNAs have a sufficient function as an initiator or a promoter.

**Example 2**

<u>Preparation of the upstream region of the synaptotagmin 11 gene</u>

DNA of the transcription control region of synaptotagmin 11 gene (hereinafter, referred to as "transcription control region DNA") was obtained by carrying out PCR using 10 ng of the human genome DNA as a template DNA, primer 1 (SEQ ID NO: 10), primer 2 (SEQ ID NO: 11) and a LA PCR Kit (Takara), The transcription control region DNA was subjected further to PCR with primer 3 (SEQ ID NO: 12) and BglII R3 Long (SEQ ID NO: 9) to ligate restriction enzyme sites thereto in order to insert it into a vector (pGL-Basic vector; Promega). The transcription control region DNA to which the restriction enzyme sites were ligated was subjected to a restriction enzyme treatment at 37°C for 1 h with MluI (Takara) and BglII (Takara) followed by applying for insertion into the vector. The pGL-Basic vector is a vector having the exact same constituent elements as the pGL3-Enhancer vector used in Example 1, except for the lack of SV 40 enhancer site.

Primer 1: 5'-GGC AGT CGA TAC TGAAAT CCA GGC-3' (SEQ ID NO: 10)

Primer 2: 5'-GAA TGG TCC TCG GAA GAA GAC TCC-3' (SEQ ID NO: 11)
Primer 3: 5'-CGA CGC GTG GCA GTC GAT ACT GAA ATC CAG-3' (SEQ ID NO: 12)

<u>Preparation of a pGL3-Basic vector containing the transcription control region DNA of the synaptotagmin 11 gene</u>

The vector used in this example was a pGL3-Basic vector (Promega). The pGL3-Basic vector that was subjected to a restriction enzyme treatment with MluI (Takara) and BglII (Takara) for 1 h at 37°C was subsequently treated three times with alkaline phosphatase (Takara) at 56°C for 1 h for dephosphorylation. The transcription control region DNA that was subjected to a restriction enzyme treatment in the same manner as above was inserted into a pGL3-Basic vector/MluI/BglII/BAP×3 using the Ligation kit (TAKARA), and subsequently transformed into *Escherichia coli* (DH5α, Invitrogen Corp.). The nucleotide sequence of the transcription control region DNA inserted into the vector was determined using an ABI PRISM 377 DNA Sequencer (Applied Biosystems), and then the vector was subjected to the reporter assay. The nucleotide sequences of transcription control regions inserted into the vectors are set forth in SEQ ID NO: 13 and SEQ ID NO: 14. It was determined that the DNA consisting of the nucleotide sequence as set forth in SEQ ID NO: 13 or SEQ ID NO: 14 contains the DNA in which the number of the sequence (SEQ ID NO: 2) units ligated is two or three, respectively. Further, the DNA consisting of the nucleotide sequence as set forth in SEQ ID NO: 3 (the number of the sequence (SEQ ID NO: 2) units ligated is two) was used instead of the transcription control region DNA and inserted into the pGL3-Basic vector by the same method. The obtained vector was then subjected to the reporter assay.

The reporter assay was carried out by the same method as described in Example 1 using the firefly luciferase gene as the reporter gene. As shown in Figure 2, the luciferase activity that is expressed in the transformants that were transformed with the above vectors increased quantitatively in proportion to the number of the sequence (SEQ ID NO: 2) units ligated within the inserted DNA having a nucleotide sequence consisting of the nucleotide sequence as set forth in SEQ ID NO: 13 or SEQ ID NO: 14. However, the luciferase activity expressed in the transformant that was transformed with the vector containing the DNA consisting of the nucleotide sequence as set forth in SEQ ID NO: 3 was low in comparison to the luciferase activity expressed in the transformant that was transfected with the DNA having a nucleotide sequence consisting of the nucleotide sequence as set forth in SEQ ID NO: 13 or SEQ ID NO: 14, but was clearly higher than that of the transformant that was transformed with the control vector.

It was thus found that, even under conditions in which the influence of a SV 40 enhancer was absent, the DNA having the sequence (SEQ ID NO: 2) unit itself exhibited transcriptional activity, and the transcriptional activity was enhanced in a manner dependent on the number of the sequence (SEQ ID NO: 2) units ligated.

Furthermore, the luciferase activity of a transformant that was transformed with a vector containing a DNA in which the number of sequence (SEQ ID NO: 2) units ligated was two was significantly higher when the vector has an SV40 enhancer (Figure 1) than when the vector does not have an SV40 enhancer (Figure 2), which showed that the transcriptional activity of the DNA is increased significantly by the influence of an SV40 enhancer.

**INDUSTRIAL APPLICABILITY**

The present invention can be used, for example, for artificially regulating the amount of expression of a gene and for producing a useful protein, and thus it is extremely useful over a wide field from basic scientific research to pharmaceutical development.

SEQUENCE FREE TEXT

SEQ ID NO: 1: Nucleotide sequence of DNA that can be used as an initiator/promoter.

SEQ ID NO: 2: Nucleotide sequence of DNA that can enhance the initiator/ promoter activity of the DNA of SEQ ID NO: 1 when being ligated to the 5' end of the same.
SEQ ID NO: 3: Nucleotide sequence of DNA that can be used as an initiator/ promoter.
SEQ ID NO: 4: Nucleotide sequence of DNA that can be used as an initiator/promoter.
SEQ ID NO: 5: Nucleotide sequence of DNA that can be used as an initiator/promoter.
SEQ ID NO: 6: Nucleotide sequence of DNA that can be used as an initiator/promoter.
SEQ ID NO: 7: Nucleotide sequence of DNA that forms a double-stranded DNA with the DNA of SEQ ID NO: 2.
SEQ ID NO: 8: Nucleotide sequence of DNA that can be used as a primer.
SEQ ID NO: 9: Nucleotide sequence of DNA that can be used as a primer.
SEQ ID NO: 10: Nucleotide sequence of DNA that can be used as a primer.
SEQ ID NO: 11: Nucleotide sequence of DNA that can be used as a primer.
SEQ ID NO: 12: Nucleotide sequence of DNA that can be used as a primer
SEQ ID NO: 13: Nucleotide sequence of a transcription regulatory region in the synaptotagmin XI gene.
SEQ ID NO: 14: Nucleotide sequence of transcription regulatory region in the synaptotagmin XI gene.

**Claims**

1.  A DNA consisting of a nucleotide sequence as set forth in SEQ ID NO: 1.

2.  A DNA in which a 5'-end of a DNA consisting of a nucleotide sequence as set forth in SEQ ID NO: 1 is added to a 3'-end of a DNA in which one or more of a DNA consisting of a nucleotide sequence as set forth in SEQ ID NO: 2 are linked to each other, wherein the link in the DNA in which one or more of the DNA consisting of the nucleotide sequence as set forth in SEQ ID NO: 2 are linked to each other is such that the 3'-end of a DNA consisting of the nucleotide sequence as set forth in SEQ ID NO: 2 is adjacent to the 5'-end of a DNA located on that 3'-side.

3.  A DNA consisting of a nucleotide sequence as set forth in any one of SEQ ID NOS: 3 to 6.

4.  The DNA according to any one of claims 1 to 3, which has a transcriptional activity, wherein the nucleotide sequence as set forth in SEQ ID NO: 2 is a sequence unit and the transcriptional activity is enhanced in a manner dependent on the number of the sequence units contained in the DNA.

5.  The DNA according to any one of claims 1 to 3, which has a transcriptional activity, wherein the nucleotide sequence as set forth in SEQ ID NO: 2 is a sequence unit and the transcriptional activity is enhanced in a manner dependent on a number of from one to seven of the sequence units contained in the DNA.

6.  An apparatus for regulating gene expression, comprising the DNA according to any one of claims 1 to 5.

7. A DNA comprising a structural gene and the DNA according to any one of claims 1 to 5, wherein the DNA according to any of claims 1 to 5 is positioned so as to enable the expression of the structural gene.

8. A vector comprising the DNA according to any one of claims 1 to 5.

9. A vector comprising the DNA according to any one of claims 1 to 5 and a DNA having an enhancer function.

10. The vector according to claim 8 or claim 9 comprising a structural gene, wherein the DNA according to any one of claims 1 to 5 is positioned so as to enable the expression of the structural gene.

11. The vector according to any one of claims 8 to 10, wherein the vector is a mammalian expression vector.

12. The vector according to any one of claims 8 to 10, wherein the vector is a virus vector.

13. The vector according to any one of claims 8 to 12, wherein the vector is a gene therapy vector.

14. A transformant which is transformed with the vector according to any one of claims 8 to 13.

15. The transformant according to claim 14, wherein the vector is transfected into a mammalian cell.

16. A method of preparing the DNA according to any one of claims 1 to 5, wherein the method comprises reacting a DNA consisting of a nucleotide sequence as set forth in SEQ ID NO: 2 with a DNA consisting of a nucleotide sequence as set forth in SEQ ID NO: 7 to form double-stranded DNAs, then ligating the prepared double-stranded DNAs to produce a conjugate, and then carrying out a polymerase chain reaction using the conjugate as a template.

17. The method of preparing the DNA according to claim 16, wherein the polymerase chain reaction is carried out using DNAs consisting of either of nucleotide sequences as set forth in SEQ ID NOS: 8 and 9.

18. A method of regulating an amount of expression of a gene, wherein the method comprises using the DNA according to any one of claims 1 to 5, the DNA according to claim 7, or the vector according to any one of claims 8 to 13.

19. A method of producing a protein, wherein the method comprises using the DNA according to any one of claims 1 to 5, the DNA according to claim 7, the vector according to any one of claims 8 to 13, or the transformant according to claim 14 or 15.

20. A reagent kit, comprising at least one member selected from the DNA according to any one of claims 1 to 5, the DNA according to claim 7, the vector according to any one of claims 8 to 13, and the transformant according to claim 14 or 15.

Figure 1

Figure 2

Figure 3

EP 1 688 490 A1

<table>
<tr><td colspan="3" align="center">INTERNATIONAL SEARCH REPORT</td><td colspan="2">International application No.<br>PCT/JP2004/016100</td></tr>
</table>

A. CLASSIFICATION OF SUBJECT MATTER
   Int.Cl⁷ C12N15/09, C12N1/15, C12N1/19, C12N1/21, C12N5/00, C12P21/02

According to International Patent Classification (IPC) or to both national classification and IPC

B. FIELDS SEARCHED

Minimum documentation searched (classification system followed by classification symbols)
   Int.Cl⁷ C12N15/09, C12N1/15, C12N1/19, C12N1/21, C12N5/00, C12P21/02

Documentation searched other than minimum documentation to the extent that such documents are included in the fields searched

Electronic data base consulted during the international search (name of data base and, where practicable, search terms used)
   CA/MEDLINE/BIOSIS/EMBASE/BIOTECHABS/WPIDS(STN),
   GenBank/EMBL/DDBJ/GeneSeq

C. DOCUMENTS CONSIDERED TO BE RELEVANT

| Category* | Citation of document, with indication, where appropriate, of the relevant passages | Relevant to claim No. |
|---|---|---|
| Y | von Poser, Christine; Ichtchenko, Konstantin; Shao, Xuguang; Rizo, Josep; Sudhof, Thomas C., The evolutionary pressure to inactivate. A subclass of synaptotagmins with an amino acid substitution that abolishes Ca2+ binding, Journal of Biological Chemistry(1997), 272(22), 14314 to 14319 | 1-20 |
| Y | Werner, Thomas, The state of the art of mammalian promoter recognition, Briefings in Bioinformatics(2003), 4(1), 22 to 30 | 1-20 |

☒ Further documents are listed in the continuation of Box C.     ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>    01 December, 2004 (01.12.04) | Date of mailing of the international search report<br>    21 December, 2004 (21.12.04) |
|---|---|
| Name and mailing address of the ISA/<br>    Japanese Patent Office | Authorized officer |
| Facsimile No. | Telephone No. |

Form PCT/ISA/210 (second sheet) (January 2004)

14

**EP 1 688 490 A1**

**INTERNATIONAL SEARCH REPORT**

International application No.

PCT/JP2004/016100

C (Continuation). DOCUMENTS CONSIDERED TO BE RELEVANT

| Category* | Citation of document, with indication, where appropriate, of the relevant passages | Relevant to claim No. |
|---|---|---|
| Y | Ohler, Uwe, Promoter Prediction on a genomic scale-the Adhexperience, Genome Research(2000), 10(4), 539 to 542 | 1-20 |

Form PCT/ISA/210 (continuation of second sheet) (January 2004)

15